# EUROPEAN PATENT APPLICATION

(11) **EP 1 728 530 A1**
(43) Date of publication of application: **06.12.2006**
(21) Application number: 05104757.9
(22) Date of filing: 01.06.2005
(51) Int. Cl.: A61M 5/32

(54) **Injection syringe with automatically retractable needle**

(71) Applicant: Medsafe ASA, 0319 Oslo (NO)
(72) Inventor: Nilsson, Peter, 553 22, Jönköping (SE); Arnerdal, Tomas, 564 35, Bankeryd (SE); Rinman, Johan, 578 92, Aneby (SE)
(74) Representative: Holme Patent A/S

(57) **Abstract**

An injection syringe is comprising a tubular housing (2), a hollow plunger (11) disposed for reciprocating displacements, partly in the housing, between an advanced and a retracted position, an elongated retraction member (14) arranged for being moved from an advanced to a retracted position in relation to the plunger with at least a part of the retraction member placed inside the plunger, and a spring (23) acting with a spring power between the plunger and the retraction member. The injection syringe (1) further comprises a hypodermic needle (10), which is retractable into the plunger, a releasable retainer arrangement (27) acting between the plunger and the retraction member for retaining the retraction member in its advanced position in relation to the plunger, a releasing means (30) for releasing the retainer when the plunger is in or close to its advanced position, and a coupling (19,34) for connecting the retraction member and the needle during or immediately after releasing the retainer.

## Description

### Field of the invention

The invention relates to an injection syringe and to a method for producing this syringe.

The injection syringe comprises a tubular housing, a hollow plunger disposed for reciprocating displacements, partly in the housing, between an advanced and a retracted position, an elongated retraction member arranged for being moved from an advanced to a retracted position in relation to the plunger with at least a part of the retraction member placed inside the plunger, and at least one spring acting with a spring power between the plunger and the retraction member.

### Background art

From the applicant's international patent application PCT/EP2004/005997, which is incorporated in the present patent application by reference, is known a disposable injection syringe comprising a piston, which is reciprocatingly mounted in a housing. The piston is connected with a piston rod for manually operating the piston. The known syringe moreover comprises a coupling for at the end of an injection stroke connecting the piston with a hypodermic injection needle protruding from the housing. The needle is then retracted into the housing by manually retracting the piston. The needle is, in retracted position, tilted in such way that it is prevented from protruding from the housing once more.

It is by means of this known disposable injection syringe effectively prevented that bacteria, which may adhere to a protruding hypodermic injection needle of an already used syringe, accidentally or by re-use infect other persons.

The U.S. Patents Nos. 5,385,551, 5,578,011 and 6,090,077 disclose disposable injection syringes arranged for automatically retracting the injection needle at the end of an injection stroke. The needle is attached to a needle holder releasable mounted in the tip of the housing of the syringe loaded by a pre-stressed spring placed in the tip too. A retainer arrangement serves to keep the needle holder in position. The injection takes place by pushing a plunger forward in the housing. The plunger is formed with an internal cavity closed by a resilient, dislodgeable stopper. The plunger activates, at the end of the injection stroke, the retainer arrangement which then releases the needle holder, after which the spring power of the spring presses the needle holder against the stopper in the plunger by the spring power of the spring. This pressure dislodges the stopper thereby allowing the needle holder with the needle to be forced into the cavity of the plunger during the expansion of the spring.

The spring of these known syringes has a relatively little diameter and a relatively short length in compressed state. The capacity of the spring is therefore very limited as regards being able to present the necessary power for forcing the needle holder with the needle together with the stopper all the way until the needle is fully retracted in the housing and/or the cavity of the plunger and the syringe therefore safely can be disposed.

Another drawback consists in that the power for dislodging the stopper tends to force the needle holder with the needle in the direction out of the hosing instead of into the housing with the risk of getting jammed in the tip of the housing whereby the desired retracting of the needle might be unreliable.

Since the plunger need to be displaced an empty distance during the dislodging of the stopper, the syringe cannot be emptied to a sufficient extent during an injection stroke. The known syringes are therefore relatively uneconomical to use.

A similar syringe is known from U.S. Patent No. 5,389,076. The spring is, in this case, acting on the stopper instead of the needle holder allowing the spring to be more appropriately dimensioned. The needle holder and the stopper are however preliminarily connected with each other thereby increasing the risk of the desired retracting of the needle being unreliable. Also this syringe suffers from the problem of not emptying the syringe sufficiently of fluid during an injection stroke.

Owing to the fact that plunger needs to be displaced an empty distance in the housing of the syringe during dislodging, the stoppers will, in the above mentioned four U.S. patents, all function with a relatively large inaccuracy when injecting a portion of fluid, e.g. medicine, into a patient.

Said prior art syringes moreover have a relatively complicated and therefore expensive construction.

The above-mentioned disadvantages of the prior art syringes are according to the present invention remedied by,
in a first aspect of the invention providing an injection syringe of the kind mentioned in the opening paragraph, with which the hypodermic needle more safely than hitherto known will be retracted automatically into the syringe at the end of an injection stroke,
in a second aspect of the invention providing an injection syringe of the kind mentioned in the opening paragraph, in which an already retracted needle is effectively secured against protruding once more from the housing of the syringe,
in a third aspect of the invention providing a ninjection syringe of the kind mentioned in the opening paragraph, in which the needle is protected against being stressed by a force acting in the opposite direction of the retraction force,
in a forth aspect of the invention providing an injection syringe of the kind mentioned in the opening paragraph, in which the fluid, e.g. medicine, can be dosed more accurately than hitherto known,
in a fifth aspect of the invention providing an injection syringe of the kind mentioned in the opening paragraph, in which the syringe can be emptied of fluid, e.g. medicine, to a larger extent than hitherto known,
in a sixth aspect of the invention providing an injection syringe of the kind mentioned in the opening paragraph, which has a simple and inexpensive construction, and
in a seventh aspect of the invention providing an injection syringe of the kind mentioned in the opening paragraph, which is well suited for mass production.

### Summary of the invention

The novel and unique features of the invention consist in the fact that the injection syringe further comprises a hypodermic needle which is displaceable between a first position, wherein it is releasable mounted in the housing and a part of the needle is protruding from the housing, and a second position, wherein the needle is retracted into the housing and/or the plunger, a releasable retainer arrangement acting between the plunger and the retraction member for retaining the retraction member in its advanced position in relation to the plunger, a releasing means for releasing the retainer when the plunger is in or close to its advanced position, and a coupling for connecting the retraction member and the needle during or immediately after releasing the retainer.
The syringe of this construction is safe, accurate and economic to use and can be disposed without any risk of other persons being infected by bacteria adhered to the needle. The syringe can moreover be mass-produced in a very economical way.

The coupling of the invention is arranged for transmitting pulling power only between the retraction member and the hypodermic needle thereby advantageously avoiding the risk of blocking the retracting of the needle at the end of the injection stroke by acting on the needle with a force in the opposite direction of the retracting direction.

According to the invention the coupling can comprise a first and second coupling part, whereby the first one can have a hook and the second one a ring arranged for allowing the hook to pass through the ring in the direction of the needle but not in the opposite direction.

The hook will at the end of an injection stroke thereby easily be able to pass the ring but is afterwards prevented from disengaging the ring whereby a traction force can be transmitted from the retraction member to the needle for retracting this into the housing and/or the hollow plunger.

In an advantageous embodiment according to the invention the hook can be arranged at the end of a protruding pin of the retraction member, and the ring can moreover be connected with a holder for the needle by means of at least one strap having a larger length than the length of the hook whereby it is effectively prevented that the needle holder with the needle can be acted on with a force in the opposite direction than the retracting direction.

According to the invention the releasing means can comprise a first releasing stop on the housing co-operating with a second releasing stop on the retraction member for preventing further advancing of the retraction member when the plunger is pushed forward in the housing to an intermediate position situated at a distance from the advanced position of the plunger. The retainer arrangement acting between the plunger and the retraction member is released when pushing the plunger further forward from said intermediate position, as the retainer arrangement is formed with a resistance against axial loadings which is lower than the reaction force acting between the first and second releasing stop when pushing the plunger forward with a predetermined power.

The retainer arrangement can, in one embodiment according to the invention, comprise a first retraining part on the plunger and a second retraining part on the retraction member, which retraining parts can be deformed in relation to each other to such an extent that they will pass each other when pushing the plunger past its intermediate position by means of sufficient power thereby releasing the retraction member.

In an appropriate embodiment of the invention the first retraining part can be equipped with at least one first projection and the second retraining part with at least one second projection, whereby the first and second projections overlap each other radially and abut against each other influenced by the spring power of the spring. The projections can be helpful for, by acting on the plunger with relatively little force, providing the necessary deformation of the first and/or the second retaining parts for releasing the retainer arrangement. Said deformation can result in the retaining parts breaking.

In a preferred embodiment of the invention the tubular housing can comprise a first tubular housing part accommodating the plunger, a second tubular housing part having a smaller diameter than the first one, and a transverse third housing part connecting the first and second housing parts.

In a simple and effective embodiment of the invention the first releasing stop can then be formed on the third housing part while the second releasing stop is an opposite shoulder on the retraction member.

According to the invention the plunger at its end portion can be equipped with a gasket for sealing the hollow plunger in relation to the first tubular housing part, which gasket advantageously can be arranged for simultaneously sealing the hollow plunger in relation to the retraction member in the advanced position of this. The gasket thereby enables the plunger to expel the fluid in the syringe through the hypodermic needle while the plunger is pushed forward from its retracted to its advanced position.

An important part of the invention consists in the gasket being able to be compressed in such way that it will abut at least a part of the inner side of the third housing part while pushing the plunger forward in the first housing part from at least the intermediate position of the plunger thereby advantageously securing that as much of the fluid in the syringe as possible is utilized. The gasket can e.g. be made of an elastomer.

The syringe of the invention can be produced in a very economical way by simultaneously injection moulding the hollow plunger and the elongated retraction member in such way that the first retraining part on the plunger and the second retraining part on the retraction member are integral moulded.

### Brief description of the drawings

Fig. 1 is a cross section along the axis of the injection syringe of the invention with the hollow plunger positioned in a starting position for the injection operation,
Fig. 2 shows the same syringe with the plunger positioned in an intermediate position in relation to the retraction member in which this are ready to be released,
Fig. 3 shows the same syringe during the releasing of the retainer arrangement,
Fig. 4 shows the same syringe with the plunger positioned in an advanced position, in which at least the major part of the fluid in the syringe has been expelled through the hypodermic needle of the syringe,
Fig. 5 shows the same syringe with the plunger positioned in a retracted position, in which the hypodermic needle is retracted into the syringe,
Fig. 6 is a cross section along the axis of a fragment of the syringe of fig. 1 - 5 showing on a greater scale a first embodiment of the retainer arrangement with the plunger positioned in the intermediate position, in which the retraction member are ready to be released, and also the coupling for coupling the retraction member and the hypodermic needle of the syringe together after the retraction member has been released,
Fig. 7 shows the fragment of fig. 6 in which the retainer arrangement is being released,
Fig. 8 shows the fragment of fig. 6 in which the retainer arrangement has been released and the retraction member and the hypodermic needle of the syringe are ready to be coupled together,
Fig. 9 shows the fragment of fig. 6 in which the retainer arrangement has been released and the retraction member and the hypodermic needle of the syringe have been coupled together,
Fig. 10 is a cross section along the axis of a fragment of the syringe of fig. 1 - 5 showing on a greater scale a second embodiment of the retainer arrangement ready to be released and the coupling for coupling the retraction member and the hypodermic needle of the syringe together after releasing of the retainer arrangement,
Fig. 11 shows the fragment of fig. 10 in which the retainer arrangement is being released, and
Fig. 12 shows the fragment of fig. 10 in which the retainer arrangement has been released and the retraction member and the hypodermic needle of the syringe are ready to be coupled together.

### Detailed description of the invention

In the description that follows it is assumed that the injection syringe of the invention is used for injecting a medicine into the body of a patient. The syringe can be a syringe prefilled with the medicine or an empty syringe which is filled with the medicine when the injection operation is to take place.

The reference number 1 refers to the syringe in general. The main parts of the syringe have, in this case, a circular cross section but may in other embodiments have other cross sections. Like parts are referred to by the same reference numerals.

The syringe comprises an elongate tubular housing 2, consisting of a first tubular housing part 3 and a second tubular housing part 4, which preferable is having a smaller diameter than the first tubular housing part. Said two housing parts are merging into each other via a traversal third housing part 5.

The second housing part 4 consists of a muff 6 extending from the transverse third housing part 5 and a cap 7 with a through hole 8 in its end portion 9 for introduction of a hypodermic needle 10 into the cap.

The muff 6 and the cap 7 could within the scope of invention be formed in any appropriate way. In practice is it, however preferred to form the muff and cap 6,7 as conical fittings with a 6% (Luer) taper according to European Standard No. EN 20594-1:1993/A1 or International Standard ISO 594-2:1998(E).

An elongate plunger 11 is disposed for reciprocating displacements in the first tubular housing part 3 with a part of the plunger projecting from said house part. Inside the plunger is formed with an elongate cavity 12 and is at the end portion of the plunger equipped with a casket 13 for sealing the plunger in relation to the first housing part 3.

An elongated retraction member 14 is arranged for being moved from an advanced to a retracted position in relation to the plunger. At least a part of the retraction member is placed in the cavity 12 of the plunger.

In this case the retraction member has a first retraction member part 15, a second retraction member part 16, a third retraction member part 17 and a fourth retraction member part 18 which at the end has a hook 19. The first retraction member part 15 has a larger diameter than the second retraction member part 16 and is merging into this via a first shoulder 20. The second retraction member part 16 has a larger diameter than inner diameter of the muff 6 and of the third retraction member part 17 and is merging into this via a second shoulder 21. The third retraction member part 17 has a larger diameter than the fourth retraction member part 18 and is merging into this via a third shoulder 22.

The gasket 13 for sealing the plunger in relation to the first tubular housing part 3 of the tubular housing 2 is arranged for also sealing the plunger in relation to the second retraction member part 16 of the retraction member 14. The gasket and the retraction member are thereby making the plunger tight for, during an injection stroke, effectively and precisely being able to expel the medicine in the syringe through the protruding hypodermic needle of the syringe in an exactly measured quantity.

The gasket is able to be compressed in such way that it will abut at least a part of the inner side of the third housing part while pushing the plunger forward in the first housing part from at least the intermediate position of the plunger thereby advantageously securing that as much of the fluid in the syringe as possible is utilized. The gasket can e.g. be made of an elastomer.

A spring 23 is acting between a collar 24 of a head 25 on the retraction member and an inwardly facing shoulder 26 on the plunger.

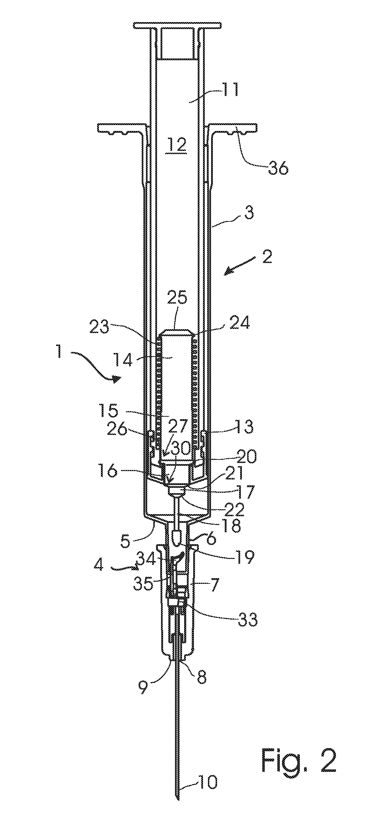

In the position of the plunger shown in fig. 2 the spring 23 is pre-stressed, that means that the spring is acting on the retraction member with a spring power trying to force the retraction member to the rear in the plunger. The retraction member is however retained in said position by means of a releasable retainer arrangement 27 consisting of a first retainer part 28 on the plunger and a second retainer part 29 on the retraction member.

A releasing means 30 is arranged for releasing the retainer arrangement when the plunger is pushed forward from the position shown in fig. 2. The releasing means comprises a first releasing stop 31 on the housing and a second releasing stop 32 on the retraction member. The first releasing stop is an inner part of the inner side of the transverse third housing part 16 and the second releasing stop is an outer part of the second shoulder 21 of the retraction member.

The hypodermic needle 10 is fastened in a needle holder 33, which is releasable mounted in the cap 7. The needle holder is at a distance from the needle holder connected to a ring 34 by means of, in this case, a single strap 35.

The hook 19 on the fourth retraction member part 18 of the elongate retraction member 14 forms a coupling together with the ring 18 as the hook and the ring are formed in such way that the hook is allowed to pass through the ring in the direction of the needle holder but not in the opposite direction.

Said coupling can transmit pulling power only between the retraction member 14 and the needle holder 33 via the strap 35 which in an advantageously embodiment cannot resist compressive forces. Moreover there still is a distance between the hook and the needle holder when the plunger and retraction member both are in their advanced positions thereby advantageously preventing that the needle holder with the needle is pushed more or less out of the cap 7 with the risk that the retracting of the needle holder is blocked.

The injection syringe of the invention is functioning in the following way.

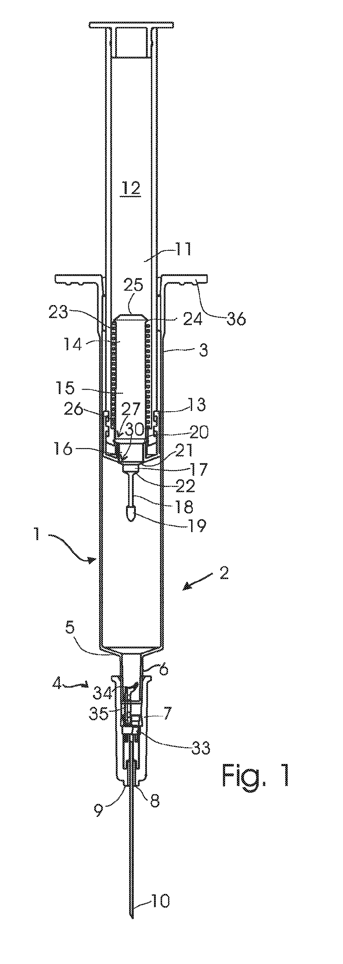

In fig. 1 the hollow plunger of the syringe is positioned in a starting position for the injection of a medicine (not shown) into a patient (not shown).

The introduction end of the first tubular housing part 3 of the tubular housing 2 is equipped with a collar 36 for holding the housing with normally two fingers (not shown) while the hollow plunger 11 is pushed into the housing by the pressure of a third finger (not shown) for thereby expelling the medicine through the hypodermic needle.

In fig. 2 the plunger has, in this way, been pushed into an intermediate position, in which the releasing means are ready to release the retainer arrangement.

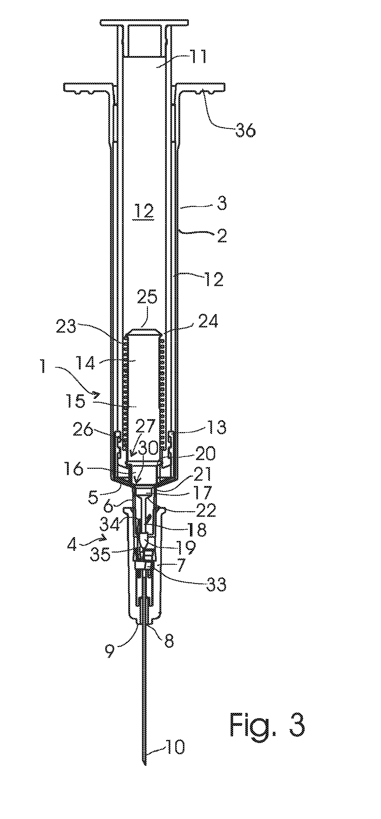

The retainer arrangement is in fig. 3 now going to be released by pushing the plunger past the intermediate position.

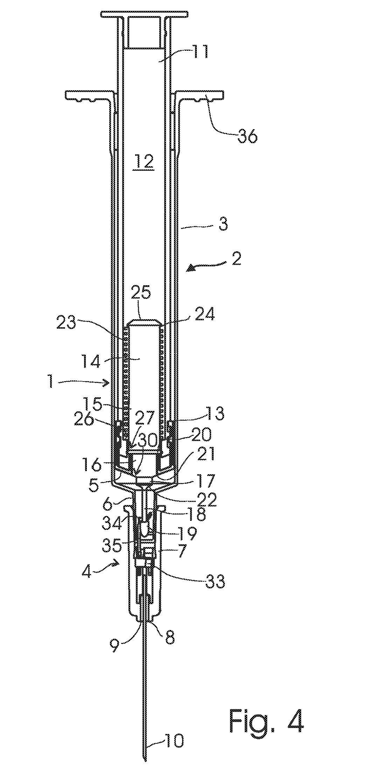

In fig. 4 the plunger has been pushed into an advanced position in which at least the major part of the fluid in the syringe has been expelled through the hypodermic needle of the syringe. The retainer arrangement has at the same time been fully released.

Releasing the retainer arrangement for retaining the elongated retraction member 14 in its advanced position in the hollow plunger 11 results in the spring power of the pre-stressed spring 23 forcing the retraction member into the hollow plunger. The needle holder will simultaneously be retracted into the hollow plunger as the retraction member and the needle holder are coupled together immediately after releasing of the retraction member.

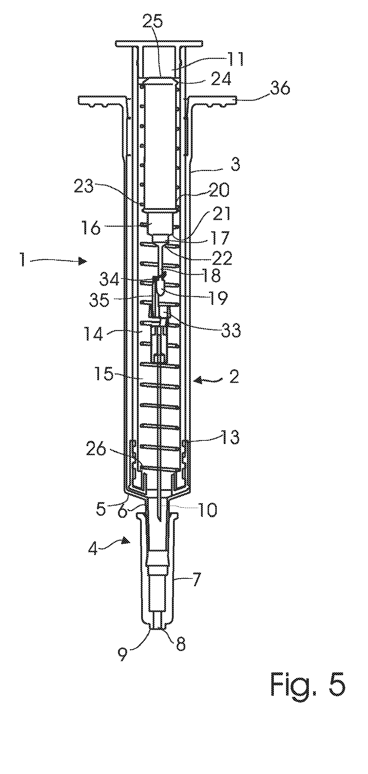

Fig. 5 shows that the needle is now fully retracted in the tubular hoses and the hollow plunger thereby effectively preventing the bacteria that may adhere to the needle after the injection has taken place from accidentally or by re-use infecting other persons.

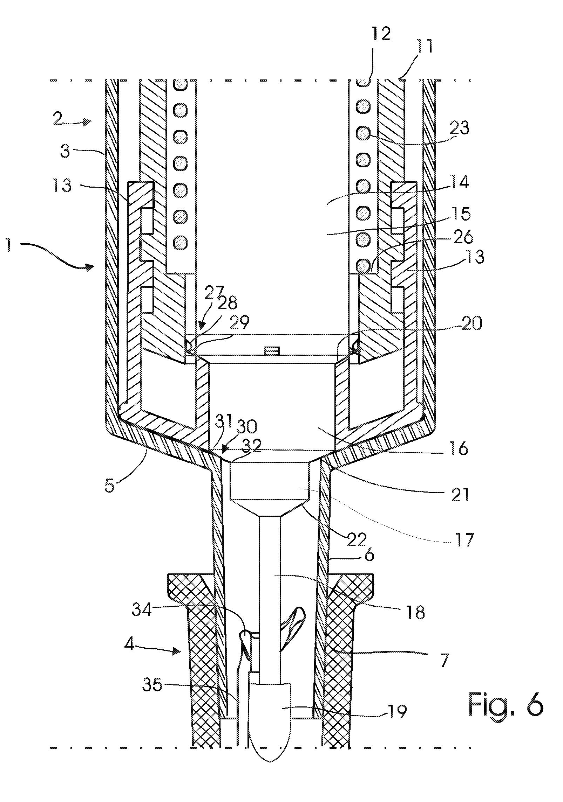

Figs. 6 - 9 show on a greater scale a fragment of the syringe of the invention with a first embodiment of the retainer arrangement of the invention.

The releasable retainer arrangement 27 consists in this case of a ring-formed first projection 28 in the hollow plunger and in this case four projections 29 on the first retraction part 14 placed close to the first shoulder 20.

It is noted that the first ring-formed projection instead can be formed on the first retraction part while the four second projections can be formed in the hollow plunger and also that the number of the second projections can be different from number of four.

In fig. 6 the plunger is in its intermediate position where the retainer arrangement is ready to be released. As can be seen the component and the second projections 29 overlap each other and furthermore abut each other influenced by the spring power of the pre-stressed spring.

The second projections 28 are arranged in such way that they can be deformed. The side of the first projection 28, abutting the four second projections 29 is furthermore inclined. Owing among other things to the fact that said side of the first projection is inclined the second projections 29 will deform when a sufficient force arises between the abutting first projection and second projections.

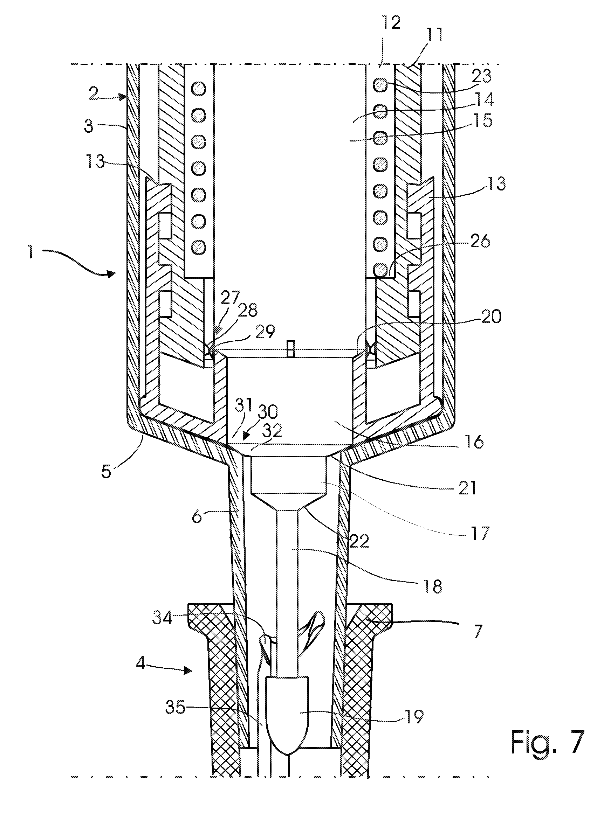

This force arises by further pushing the plunger a little distance into the housing from the intermediate position shown in fig. 6 with a predetermined force since the second shoulder 21 of the retraction member 14 is engaging the transverse third housing part 5 in the intermediate position of the plunger thereby stopping further forward movement of the retraction member. The second projections 29 will then deform as seen in fig. 7.

The second projections are in this deformed position able to pass the first projection and thereby release the retraction member.

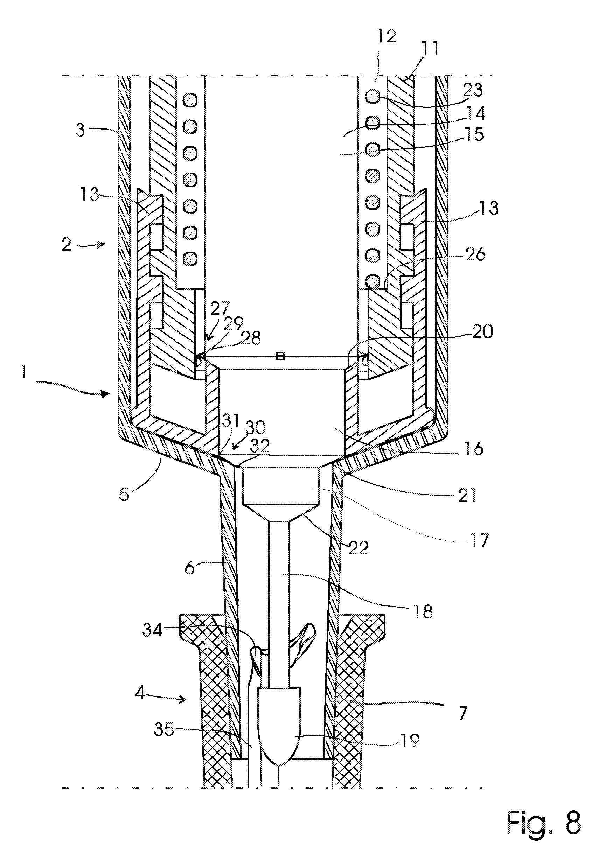

Fig. 8 shows the position of the retraction member immediately after the releasing of the retraction member where the hook 19 has now passed the ring 34 and the retraction member is ready to be retracted into the tubular housing and the hollow plunger influenced by the spring power of the spring.

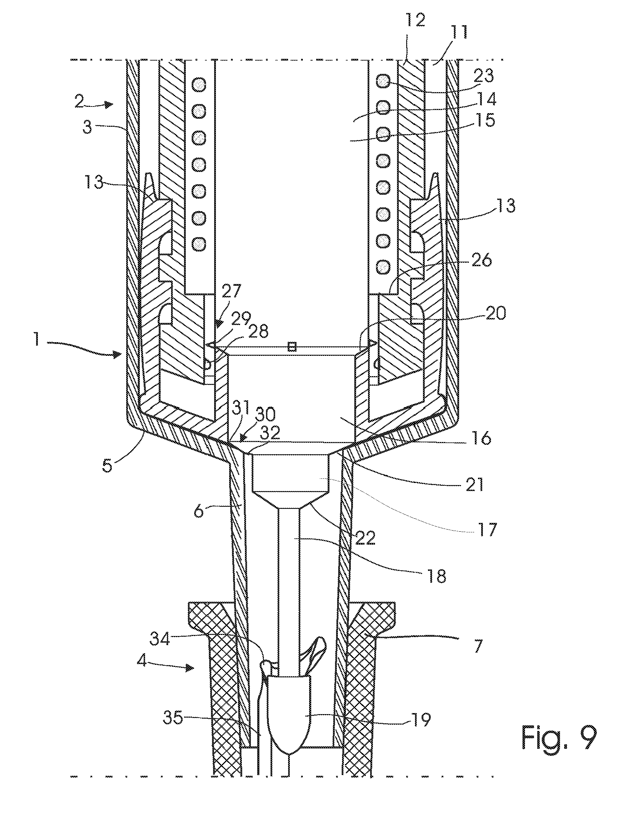

Fig. 9 shows the retraction member retracted so much into the hollow plunger that the hook and the ring couple together.

The retraction member and the needle holder are now connected in such way that the spring power of the spring acting on the retraction member will be transmitted to the hypodermic needle holder with the needle via the active coupling between the retraction member and the needle holder.

The needle holder with the hypodermic needle will therefore, during the continued retracting process, be pulled completely into the tubular housing and the hollow plunger. This situation is shown in fig. 5.

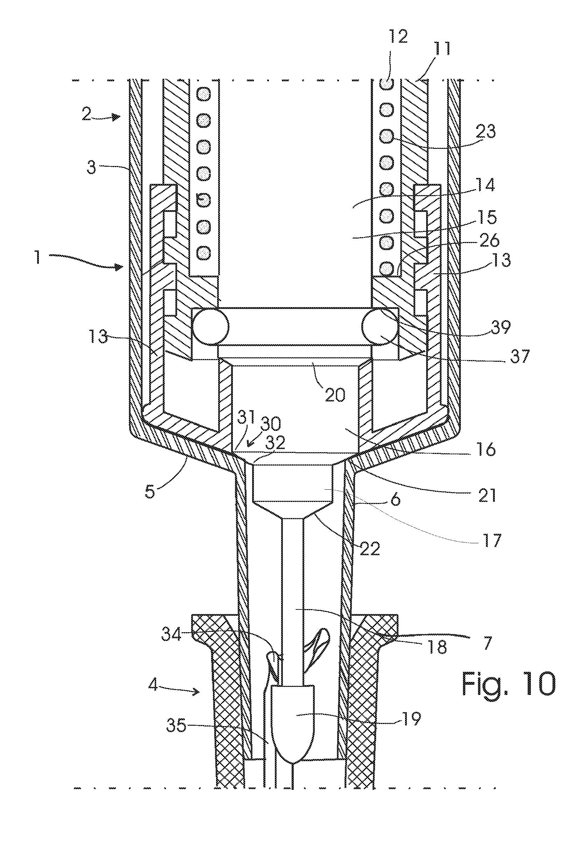

Figs. 10 - 12 show on a greater scale a fragment of the syringe of the invention with a second embodiment of the retainer arrangement of the invention.

In fig. 10 the plunger is in its intermediate position where the retainer arrangement is ready to be released. As can be seen, the releasable retainer arrangement consists of an elastic O-ring 37 placed in a groove 38 in the retraction member 14. A shoulder 39 on the hollow plunger 11 is simultaneously resting on the O-ring.

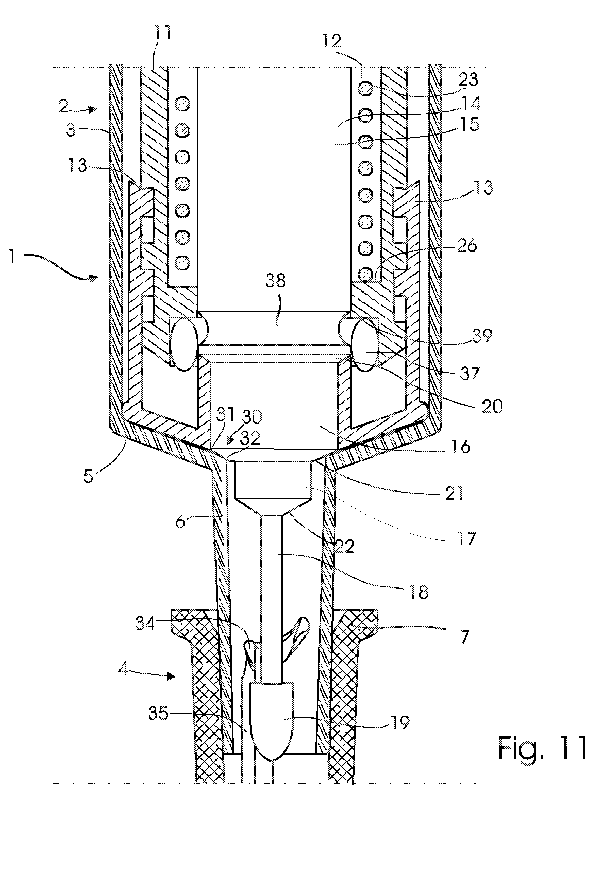

The hollow plunger will, when acted on by a predetermined force, be pushed forward a little distance further in the housing resulting in the shoulder 38 on the plunger forcing the O-ring 37 to leave the groove 38 in the retraction member 14, because the second shoulder 21 of the retraction member 14 is engaging the transverse third housing part 5 thereby stopping further forward movement of the retraction member. This situation is shown on fig. 11

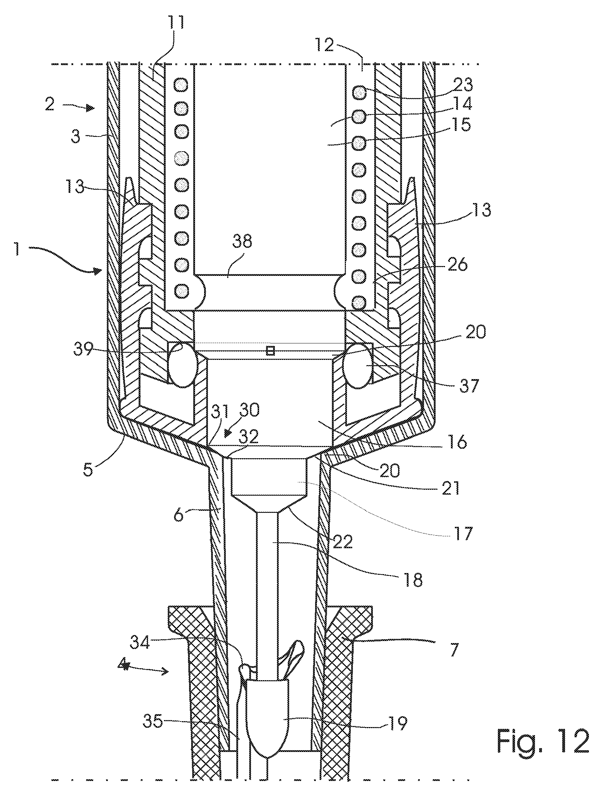

Fig. 12 shows the position of the retraction member immediately after the releasing of the retraction member where the hook 19 has passed the ring 34. The retraction member will then be retracted into the housing influenced by the spring power of the spring. The needle holder with the hypodermic needle will, during the continued retracting process, be coupled together with the retraction member and by this be pulled completely into the tubular housing and/or the hollow plunger.

## Claims

1. An injection syringe comprising
- a tubular housing (2),
- a hollow plunger (11) disposed for reciprocating displacements, partly in the housing, between an advanced and a retracted position,
- an elongated retraction member (14) arranged for being moved from an advanced to a retracted position in relation to the plunger with at least a part of the retraction member placed inside the plunger, and
- at least one spring (23) acting with a spring power between the plunger and the retraction member,
**characterized in that** the injection syringe (1) further comprises
- a hypodermic needle (10) which is displaceable between a first position, wherein it is releasable mounted in the housing and a part of the needle is protruding from the housing, and a second position, wherein the needle is retracted into the housing and/or the plunger,
- a releasable retainer arrangement (27) acting between the plunger and the retraction member for retaining the retraction member in its advanced position in relation to the plunger,
- a releasing means (30) for releasing the retainer arrangement when the plunger is in or close to its advanced position, and
- a coupling (19,34) for connecting the retraction member and the needle during or immediately after releasing of the retainer arrangement.

2. An injection syringe according to claim 1, **characterized in that** the coupling (18,19;34,35) comprises a first coupling part (18,19) on the retraction member (14) and a second coupling part (34,35) on the needle (10).

3. An injection syringe according to claim 2, **characterized in that** the first coupling part (18,19) comprises a hook (19) and the second coupling part (34,35) a ring (34) arranged for allowing the hook to pass through in the direction of the needle but not in the opposite direction.

4. An injection syringe according to claim 3, **characterized in that** the hook (19) is arranged at the end of a protruding pin (18) of the retraction member (14) and that the ring (34) is connected with the needle (10) or a holder (33) for the needle by means of at least one strap (35) having a larger length than the length of the hook (19).

5. An injection syringe according to any of the claims 1 - 4, **characterized in that** the releasing means (30) comprises a first releasing stop (31) on the housing co-operating with a second releasing stop (32) on the retraction member to prevent further advancing of the retraction member when the plunger is pushed forward in the housing to an intermediate position situated at a distance from the advanced position of the plunger, and that the retainer arrangement (27) is formed with a resistance against axial loadings which is lower than the reaction force acting between the first and second stop when pushing the plunger forward from said intermediate position with a predetermined force.

6. An injection syringe according to any of the claims 1 - 5, **characterized in that** the retainer arrangement (27) comprises a first retaining part (28) on the plunger and a second retaining part (29) on the retraction member.

7. An injection syringe according to claim 6, **characterized in that** the first and/or second retaining parts (28;29) are deformable to such an extent that they will be able to pass each other.

8. An injection syringe according to claim 6 or 7, **characterized in that** the first retaining part is equipped with at least one first projection (28) and the second retaining part with at least one second projection (29), that the at least one first and second projections overlap each other radially, and that they are abutting each other influenced by the spring power of the at least one spring (23).

9. An injection syringe according to claim 8, **characterized in that** the at least first and/or the at least second projections (28;29) are breakable.

10. An injection syringe according to any of the claims 1 - 9, **characterized in that** the tubular housing (2) comprises a first tubular housing part (3) accommodating the plunger (11), a second tubular housing part (4) having a smaller diameter than the first one, and a transverse third housing part (5) connecting the first and second housing parts.

11. An injection syringe according to claim 10, **characterized in that** the first releasing stop (31) is formed on the third housing part (5) and that the second releasing stop (32) is an opposite shoulder on the retraction member (14).

12. An injection syringe according to claim 10 or 11, **characterized in that** the plunger (11) at its end portion is equipped with a gasket (13) for sealing the plunger in relation to the first tubular housing part (3).

13. An injection syringe according to claim 12, **characterized in that** the gasket (13) is arranged for, in the advanced position of the retraction member (14), sealing the retraction member in relation to the plunger (14) simultaneously with sealing the plunger in relation to the first tubular housing part (3).

14. An injection syringe according to claim 13, **characterized in that** the gasket (13) is compressible, e.g. made of an elastomer, and is arranged for abutting at least part of the inner side of the third housing part (5) while pushing the plunger forward in the first housing part (3) from at least the intermediate position of the plunger.

15. A method for producing the injection syringe according to claims 1 - 14, **characterized in that** hollow plunger (11) and the elongated retraction member (14) are injection-moulded simultaneously in such way that the first retaining part on the plunger and the second retaining part on the retraction member are integrally moulded.
